# FASCICULE DE BREVET EUROPEEN

(11) **EP 3 314 047 B1**
(45) Date de publication et mention de la délivrance du brevet: **21.08.2024**
(21) Numéro de dépôt: 15747837.1
(22) Date de dépôt: 26.06.2015
(51) Int. Cl.: D04B 1/18, A61F 13/08

(54) **ARTICLE TEXTILE À POCHE, SYSTÈME ET SON PROCÉDÉ DE FABRICATION**
TEXTILARTIKEL MIT EINER TASCHE, SYSTEM UND VERFAHREN ZU SEINER HERSTELLUNG
FABRIC ARTICLE WITH A POCKET, SYSTEM AND METHOD OF MAKING SAME

(43) Date de publication de la demande: 02.05.2018
(73) Titulaire: SIMP ORTHO SOLUTIONS, 69230 Saint-Genis-Laval (FR)
(72) Inventeur: FRENAY, Patrick, Michel, Eric, F-69340 Francheville (FR); REYMOND, Jean-Louis, F-42260 Saint Germain Laval (FR)
(74) Mandataire: Cabinet Didier Martin
(86) Numéro de dépôt international: PCT/FR2015/051739
(87) Numéro de publication internationale: WO 2016/207494

(56) Documents cités:
- WO-A1-00/14316
- WO-A2-2007/056748
- FR-A1- 2 781 817
- US-A- 5 814 003

## Description

### DOMAINE TECHNIQUE

La présente invention se rapporte au domaine technique général des articles textiles destinés à entourer un segment corporel humain ou animal, comme par exemple une structure articulaire, musculaire ou osseuse, afin d'assurer une fonction thérapeutique (en particulier de nature orthopédique) et/ou non thérapeutique, par exemple une fonction de contention, de compression, de maintien, de stabilisation, de maintien au chaud ou de réchauffement.

La présente invention concerne plus précisément un article textile comprenant un premier panneau destiné à entourer une partie d'un corps humain ou animal, ledit premier panneau étant constitué par un premier tricot qui est composé de mailles formées par au moins un premier fil et qui inclut un tramage élastique pour permettre audit article d'enserrer élastiquement ladite partie du corps humain ou animal.

L'invention concerne également un procédé de fabrication d'un article textile comprenant une étape de fabrication d'un premier panneau, par tricotage d'au moins un premier fil avec insertion d'un tramage élastique, ledit premier panneau étant destiné à enserrer élastiquement une partie d'un corps humain ou animal.

### TECHNIQUE ANTERIEURE

Il est connu d'avoir recours à des orthèses pour entourer une structure articulaire, musculaire ou osseuse du corps humain, en vue d'obtenir un effet thérapeutique par une action mécanique externe, comme une action de contention, de compression, ou de maintien, par exemple à des fins de stabilisation ou d'assistance de la structure en question pendant une phase de rééducation ou de convalescence. Il existe bien entendu une multiplicité d'orthèses, chacune adaptée à une région corporelle spécifique (orthèse de poignet, genouillère, coudière, chevillière, ceinture dorso-lombaire, collier cervical...).

Il a par ailleurs été proposé d'équiper certaines de ces orthèses connues d'une poche destinée à recevoir une compresse thermo-thérapeutique, généralement désignée par l'appellation « *pack gel chaud*/*froid* », afin notamment de réduire l'inflammation et le gonflement de la zone corporelle concernée et/ou de soulager la douleur. Plus précisément, ces orthèses à poche connues sont formées d'une bande de tissu élastique destinée à venir enserrer la partie du corps humain concernée. Sur une face de cette bande est rapporté un panneau en élasthanne cousu à sa périphérie à la bande élastique pour former la poche, avec une ouverture permettant d'insérer et d'extraire à volonté un pack gel.

De telles orthèses à poche pour pack gel chaud/froid donnent globalement satisfaction, mais n'en présentent pas moins de sérieux inconvénients.

Ces orthèses à poche peuvent en effet s'avérer inconfortables, du fait de la présence des coutures de fixation du panneau en élasthanne, et leur fabrication est relativement compliquée et onéreuse, du fait de la multiplicité des opérations requises et des différents tissus techniques utilisés. Par ailleurs, la présence du panneau en élasthanne peut être de nature à affecter localement les propriétés de compression et de contention de la bande élastique. En outre, ces orthèses connues ne permettent pas en pratique un échange thermique optimal entre le pack gel inséré dans la poche et la zone corporelle enserrée par l'orthèse. La conception et la construction de ces orthèses connues n'est également pas de nature à limiter de façon optimale la déperdition thermique, de sorte qu'une partie des calories ou frigories dispensées par le pack gel sont susceptibles d'être dissipées vers l'extérieur, à travers la bande élastique. Enfin, il existe avec ces orthèses connues un risque que le pack gel puisse s'échapper de façon intempestive hors de la poche, de sorte que le port de ces orthèses connues peut être incompatible avec une pratique sportive.

Le document FR-2 781 817 A1 divulgue en résumé un tricot tramé comportant une poche obtenue par un procédé consistant, dans une première phase, à réaliser une première rangée de mailles avec un fil qui, dans la zone de poche, travaille avec toutes les aiguilles de la fonture avant et seulement avec au moins une aiguille sur deux de la fonture arrière en laissant des aiguilles libres, dans une seconde phase, à mettre en place le fil de trame sur la première rangée de mailles, dans une troisième phase, dans la zone de la poche, à prélever, avec un rapport d'au moins 1/2 une maille de la fonture arrière pour la reporter sur une aiguille maillée de la fonture avant, et, dans une quatrième phase, à réaliser une deuxième rangée de mailles dont le fil travaille dans la zone de la poche, avec les seules aiguilles de la fonture arrière qui n'ont pas travaillé dans la première phase.

### EXPOSE DE L'INVENTION

La présente invention vise en conséquence à porter remède aux différents inconvénients énumérés précédemment, et à proposer un nouvel article textile destiné à entourer une partie d'un corps humain ou animal qui, tout en étant équipé d'un logement permettant d'accueillir un élément fonctionnel indépendant (comme par exemple une compresse thermique), est particulièrement confortable pour le porteur, robuste, esthétique, et présente des propriétés d'extensibilité élastique maîtrisées.

Un autre objet de l'invention vise à proposer un nouvel article textile destiné à entourer une partie d'un corps humain ou animal qui se prête particulièrement bien à une fabrication industrielle.

Un autre objet de l'invention vise à proposer un nouvel article textile destiné à entourer une partie d'un corps humain ou animal, dont la construction permet d'optimiser l'action de l'élément fonctionnel indépendant inséré dans un logement dont est pourvu ledit article textile.

Un autre objet de l'invention vise à proposer un nouvel article textile destiné à entourer une partie d'un corps humain ou animal, dont la construction est particulièrement simple et peut être mise en oeuvre de façon mécanisée au moyen d'un matériel industriel classique.

Un autre objet de l'invention vise à proposer un nouvel article textile destiné à entourer une partie d'un corps humain ou animal, qui est pourvu d'un logement conçu pour maintenir en son sein de manière particulièrement efficace un élément fonctionnel indépendant, tel qu'une compresse thermique, sans risque de voir ledit élément fonctionnel s'échapper intempestivement hors dudit logement.

Un autre objet de l'invention vise à proposer un nouvel article textile destiné à entourer une partie d'un corps humain ou animal, dont la fabrication repose sur la mise en oeuvre d'opérations élémentaires particulièrement simples.

Un autre objet de l'invention vise à proposer un nouveau procédé de fabrication d'un article textile destiné à entourer une partie d'un corps humain ou animal, qui permet d'obtenir de façon particulièrement simple, rapide et bon marché un article textile qui tout en étant muni d'un logement capable d'accueillir de façon amovible un élément fonctionnel indépendant, est particulièrement confortable et robuste.

Un autre objet de l'invention vise à proposer un nouveau procédé de fabrication d'un article textile destiné à entourer une partie d'un corps humain ou animal, qui peut être aisément automatisé.

Un autre objet de l'invention vise à proposer un nouveau procédé de fabrication d'un article textile destiné à entourer une partie d'un corps humain ou animal, qui repose sur la mise en oeuvre d'opérations élémentaires simples, à partir d'un minimum de composants de départ différents.

Un autre objet de l'invention vise à proposer un nouveau procédé de fabrication d'un article textile destiné à entourer une partie d'un corps humain ou animal, qui peut être mis en oeuvre de façon mécanisée au moyen d'un matériel industriel standard.

Les objets assignés à l'invention sont atteints à l'aide d'un article textile comprenant un premier panneau destiné à entourer une partie d'un corps humain ou animal, ledit premier panneau étant constitué par un premier tricot qui est composé de mailles et qui inclut un tramage élastique pour permettre audit article d'enserrer élastiquement ladite partie du corps humain ou animal, et comprenant un deuxième panneau superposé localement audit premier panneau et attaché à ce dernier pour former avec ledit premier panneau un logement qui est muni d'une ouverture d'accès afin d'accueillir de façon amovible en son sein un élément fonctionnel indépendant, ledit logement (6) constituant une poche formée par un panneau avant et un panneau arrière (7) mutuellement attachés en périphérie, ledit panneau avant étant formé par ledit deuxième panneau (5), tandis que ledit premier panneau (2) est formé par ledit panneau arrière (7) ainsi que par un panneau principal (8) qui entoure au moins en partie ledit panneau arrière (7), les mailles dudit premier tricot qui constituent ledit panneau principal (8) étant formées par un premier fil (3) ainsi que par un deuxième fil (9), tandis que les mailles du premier tricot qui constituent ledit panneau arrière (7) sont formées par ledit deuxième fil (9), ledit deuxième panneau étant constitué par un deuxième tricot composé de mailles formées par ledit premier fil, ledit deuxième panneau (5) comprenant un bord libre (5A) qui s'étend longitudinalement entre deux extrémités (50, 51) attachées audit premier panneau (2), ledit bord libre (5A) délimitant, avec le premier panneau (2), ladite ouverture d'accès (60), ledit tramage élastique étant formé d'au moins un fil élastique (4) qui est également associé audit bord libre (5A) pour conférer à ce dernier des propriétés d'élasticité longitudinale, ledit fil élastique (4) du tramage élastique du premier panneau (2) étant utilisé pour contraindre élastiquement le bord libre (5A), tout en étant par ailleurs absent du reste du deuxième panneau (5).

Les objets assignés à l'invention sont également atteints à l'aide d'un système comprenant d'une part un article textile selon l'invention, et d'autre part un élément fonctionnel indépendant destiné à être accueilli de façon amovible au sein du logement dudit article textile, ledit élément fonctionnel faisant partie du groupe suivant : compresse thermique, dispositif de diffusion d'une substance active, comme par exemple une substance antalgique, dispositif de compression à volume variable.

Les objets assignés à l'invention sont enfin atteints à l'aide d'un procédé de fabrication d'un article textile comprenant une étape de fabrication d'un premier panneau par tricotage d'au moins un premier fil avec insertion d'un tramage élastique, ledit premier panneau étant destiné à enserrer élastiquement une partie d'un corps humain ou animal, ledit procédé étant caractérisé en ce qu'il comprend une étape de fabrication, par tricotage dudit premier fil, d'un deuxième panneau superposé localement audit premier panneau et attaché à ce dernier pour former avec ledit premier panneau un logement qui est muni d'une ouverture d'accès afin d'accueillir de façon amovible en son sein un élément fonctionnel indépendant, ledit logement (6) constituant une poche formée par un panneau avant et un panneau arrière (7) mutuellement attachés en périphérie, ledit panneau avant étant formé par ledit deuxième panneau (5), tandis que ledit premier panneau (2) est formé par ledit panneau arrière (7) ainsi que par un panneau principal (8) qui entoure au moins en partie ledit panneau arrière (7), ladite étape de fabrication (i) dudit premier panneau (2) comprenant: - une opération de fabrication dudit panneau principal (8) par tricotage dudit premier fil (3) et d'un deuxième fil (9), - et une opération de fabrication dudit panneau arrière (7) par tricotage dudit deuxième fil (9), ledit deuxième panneau (5) comprenant un bord libre (5A) qui s'étend longitudinalement entre deux extrémités (50, 51) attachées audit premier panneau (2), ledit bord libre (5A) délimitant, avec le premier panneau (2), ladite ouverture d'accès (60), ledit tramage élastique étant formé d'au moins un fil élastique (4), ledit procédé comprenant une étape d'association dudit fil élastique (4) audit bord libre (5A) pour conférer à ce dernier des propriétés d'élasticité longitudinale, ledit fil élastique (4) du tramage élastique du premier panneau (2) étant utilisé pour contraindre élastiquement le bord libre (5A), tout en étant par ailleurs absent du reste du deuxième panneau (5).

### DESCRIPTIF SOMMAIRE DES DESSINS

D'autres objets et avantages particuliers de l'invention apparaîtront plus en détails à la lecture de la description qui suit, ainsi qu'à l'aide des dessins annexés ci-après, à titre purement illustratif et non limitatif, dans lesquels :
- la figure 1 illustre, selon une vue schématique en perspective, un article textile tubulaire selon l'invention, forme en l'occurrence un manchon élastique destiné à enserrer une partie du corps humain (coude, genou ou poignet par exemple), et qui est pourvu sur sa face interne d'un logement destiné à accueillir de façon amovible en son sein un élément fonctionnel indépendant, formé par exemple par une compresse thermique du type sachet rempli d'un gel eutectique.
- la figure 2 illustre, selon une vue de face, une bande textile conçue pour former, par assemblage mutuel de ses extrémités latérales, l'article tubulaire de la figure 1.
- la figure 3 illustre, selon une vue de dos, la bande de la figure 2.
- la figure 4 illustre, selon une vue schématique en perspective de dessus, un détail de réalisation de l'article textile tubulaire de la figure 1.
- la figure 5 illustre schématiquement et de façon agrandie une portion du premier tricot formant le premier panneau de l'article textile illustré aux figures précédentes.

### MEILLEURE MANIERE DE REALISER L'INVENTION

L'invention concerne un article textile 1 destiné à entourer une partie d'un corps humain ou animal, à des fins thérapeutiques ou non-thérapeutiques. Avantageusement, l'article textile 1 est un article de contention, de compression, de maintien ou de stabilisation d'une partie d'un corps humain ou animal, laquelle est formée par exemple par une structure articulaire, musculaire ou osseuse, afin d'obtenir avantageusement un effet thérapeutique (ou préventif) par une action mécanique d'enserrement du segment corporel concerné, que ce soit par exemple en phase traumatique aiguë, au cours d'un processus de rééducation ou de convalescence (par exemple post-traumatique), ou pour prévenir un éventuel traumatisme ou une affection. L'article textile 1 selon l'invention forme ainsi avantageusement une orthèse, et en particulier une orthèse orthopédique comme par exemple une orthèse poignet/pouce, une orthèse poignet/main, un collier cervical, une ceinture lombaire, une coudière, une genouillère, *etc.* L'invention n'est cependant pas limitée à un article textile 1 thérapeutique visant à entourer une partie d'un corps humain ou animal en prévention ou en traitement d'un traumatisme, en particulier orthopédique. L'article textile 1 peut par exemple être conçu pour entourer une partie d'un corps humain ou animal en prévention ou en traitement de maux d'origine rhumatismale. L'article textile 1 consiste par conséquent avantageusement en un article textile, de préférence orthopédique, conçu pour entourer une partie d'un corps humain ou animal (faisant par exemple partie de l'appareil locomoteur) dans le cadre d'un traitement préventif ou curatif de maux d'origine traumatique ou rhumatismale et des douleurs associées.

L'invention n'est toutefois pas limitée à un article thérapeutique. L'article textile 1 peut par exemple constituer un article textile 1 de confort, et se présenter par exemple sous la forme d'une « *ceinture de chaleur*» visant à maintenir au chaud la partie du corps humain ou animal concernée, sans but curatif ou préventif, ou encore par exemple sous la forme d'une « *ceinture de grossesse* » destiné à améliorer le confort de la femme enceinte en soutenant son abdomen.

De manière générale, l'article textile 1 est donc un article textile thérapeutique (préventif ou curatif de maux d'origine traumatique ou organique) et/ou de confort, dont l'effet premier est basé essentiellement sur un enserrement élastique de la partie du corps humain ou animal à traiter.

Selon une première variante, l'article textile 1 est conçu pour être porté par une personne humaine, afin d'entourer une partie du corps de la personne en question. Il est cependant parfaitement envisageable, selon une deuxième variante, que l'article textile 1 soit conçu pour être porté par un animal, par exemple un cheval, un chien, un chat, *etc.*

L'invention concerne également un procédé de fabrication d'un tel article textile 1, de sorte que la description qui suit est applicable indifféremment à l'article 1 lui-même mais également au procédé de fabrication correspondant, et réciproquement.

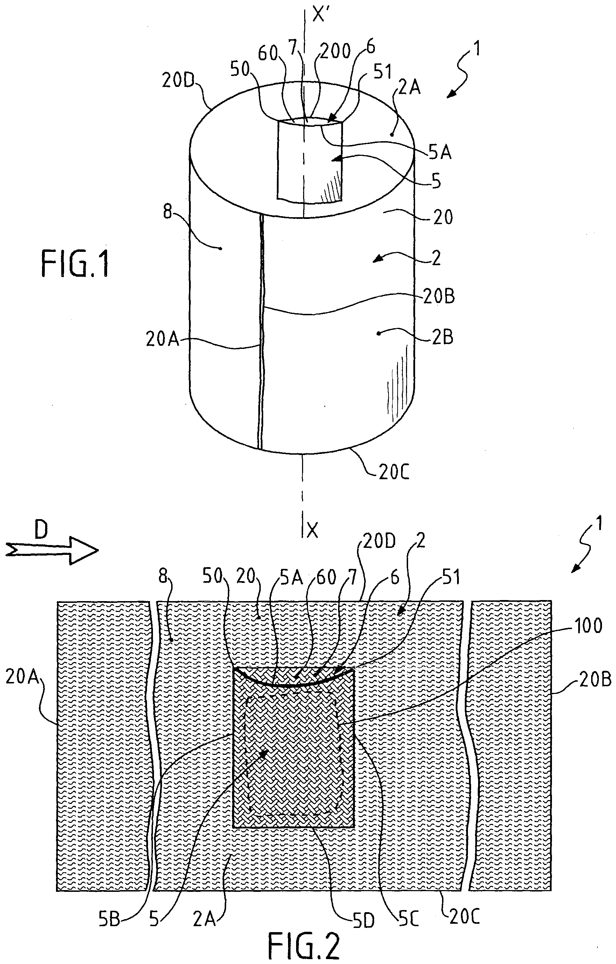

Comme illustré aux figures, l'article textile 1 selon l'invention comprend un premier panneau 2 destiné à entourer ladite partie du corps humain. A cette fin, le premier panneau 2 présente avantageusement une forme sensiblement tubulaire, de façon permanente ou temporaire. Comme illustré aux figures, le premier panneau 2 présente une face interne 2A destinée à se trouver en regard de ladite partie du corps humain à enserrer élastiquement ainsi qu'une face externe 2B opposée tournée vers l'extérieur. La face interne 2A est avantageusement destinée à venir directement au contact de la partie du corps humain à entourer. Dans le mode de réalisation illustré à la figure 1, le premier panneau 2 présente, de façon permanente, une forme tubulaire d'axe central X-X'. Dans ce cas, le premier panneau 2 forme un manchon destiné à être enfilé sur la partie du corps concernée (par exemple la cheville, le genou ou le coude...) par exemple pour assurer une contention, une compression un maintien ou une stabilisation de la partie du corps en question. Il est cependant tout à fait envisageable, sans pour autant que l'on sorte du cadre de l'invention, que le premier panneau 2 soit pourvu de moyens de verrouillage réversibles lui permettant d'évoluer entre une configuration ouverte, dans laquelle le premier panneau 2 se présente sous la forme d'une bande sensiblement plate, et une configuration fermée, dans laquelle le premier panneau 2 se présente sous la forme d'une gaine capable de ceindre la partie du corps concernée. Les moyens de verrouillage en question sont par exemple formés par un système de type velours/crochets (comme le système connu sous la marque déposée Velcro ^{®}).

Ainsi, le premier panneau 2 se présente avantageusement sous la forme d'une bande 20, qui s'étend par exemple longitudinalement entre un premier bord latéral 20A et un deuxième bord latéral 20B (de préférence parallèles audit axe vertical central X-X'), et transversalement entre un premier bord longitudinal 20C et un deuxième bord longitudinal 20D. Ladite bande 20 est en l'espèce refermée sur elle-même, soit de façon permanente comme illustré à la figure 1 (auquel cas les premier et deuxième bords longitudinaux 20A, 20B sont par exemple attachés l'un à l'autre sensiblement continûment sur toute leur hauteur, par exemple au moyen d'une couture, par collage ou tout autre moyen) soit de façon temporaire (auquel cas les premier et deuxième bords latéraux 20A, 20B sont pourvus de moyens de verrouillage complémentaires réversibles, par exemple du genre velours/crochets comme exposé précédemment), pour entourer ladite partie du corps humain. Dans l'exemple illustré aux figures, la bande 20 présente sensiblement une forme rectangulaire, dont les bords latéraux 20A, 20B et longitudinaux 20C, 20D sont rectilignes et parallèles deux à deux. Il est cependant parfaitement envisageable, sans pour autant que l'on sorte du cadre de l'invention, que les bords latéraux 20A, 20B et/ou longitudinaux 20C, 20D soit sensiblement courbes, de façon que l'article textile 1 présente une conformation parfaitement adaptée au segment corporel qu'il est destiné à enserrer.

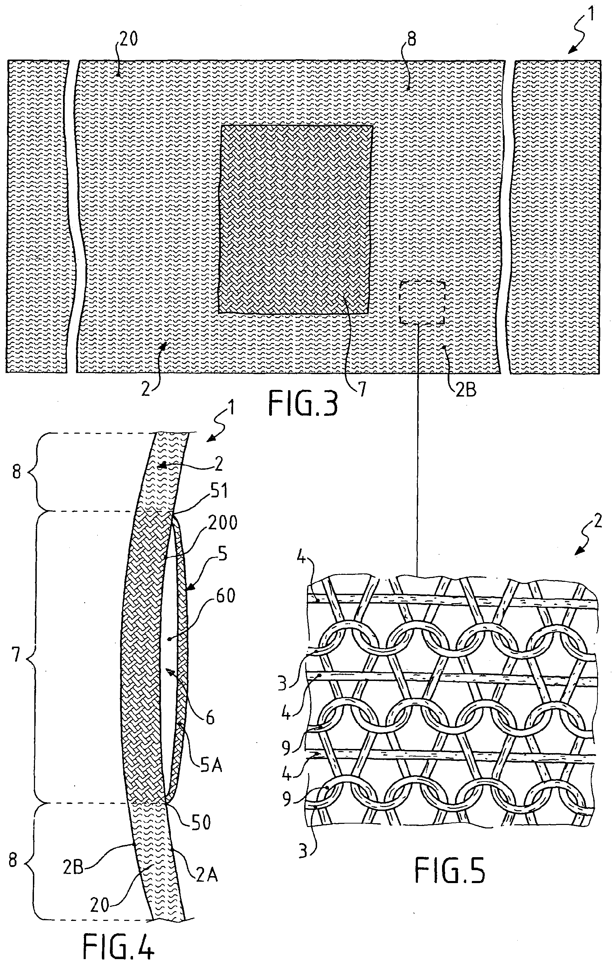

Conformément à l'invention, le premier panneau 2 est constitué par un premier tricot, c'est-à-dire par une étoffe à mailles, de préférence à mailles cueillies, de sorte que le premier tricot formant le premier panneau 2 est avantageusement un tricot en trame, c'est-à-dire un tricot à mailles cueillies, dont les mailles sont disposées en rangs et en colonnes (cf. figure 5). Ledit premier tricot formant le premier panneau 2 est composé de mailles elles-mêmes formées par au moins un premier fil 3, lequel peut être un fil naturel (coton par exemple) ou synthétique (polyamide ou polyester par exemple). Ledit premier tricot inclut en outre un tramage élastique pour permettre audit article 1 d'enserrer élastiquement ladite partie du corps humain. Le tramage élastique en question est formé d'au moins un fil élastique 4, qui est non tricoté (c'est à dire qui ne forme pas de mailles, contrairement audit premier fil 3), et qui s'étend de façon sensiblement rectiligne au sein du premier tricot, formant ainsi des « flottés » élastiques capturés au sein de la structure de mailles et destinés à impartir un comportement élastique spécifique et maîtrisé audit premier tricot.

Le procédé de fabrication selon l'invention comprend donc une étape (i) de fabrication d'un premier panneau 2, par tricotage d'au moins un premier fil 3 avec insertion d'un tramage élastique afin de permettre audit premier panneau 2 de pouvoir enserrer élastiquement la partie du corps humain à traiter. Comme exposé précédemment, l'étape (i) de fabrication du premier panneau 2 consiste avantageusement à tricoter en trame au moins le premier fil 3, ladite étape (i) étant avantageusement effectuée au moyen d'une tricoteuse industrielle, par exemple une tricoteuse industrielle rectiligne, de préférence à deux fontures au moins, capable de tricoter des bandes à plat.

Les trames élastiques formant ledit tramage élastique inséré au sein dudit premier tricot sont par exemple formées à partir d'un ou plusieurs fils 4 en élasthanne ou en élastodiène, éventuellement guipés (par exemple avec un guipage coton). De préférence, les trames élastiques insérées dans le premier tricot, pour former ledit tramage élastique, sont formés par un seul et même fil élastique 4 continu déposé selon un trajet en méandres, par étages successifs reliés par des virages en lacets, la longueur des trames élastiques en question étant dans ce cas conjuguée à celle des rangs de mailles. Par exemple, comme illustré à la figure 5, chaque rang de mailles du premier tricot est associé à une trame élastique de longueur sensiblement identique à celle dudit rang. Le tramage élastique mis en oeuvre dans le premier tricot permet ainsi au premier panneau 2 d'étreindre élastiquement la partie du corps humain concernée, afin par exemple d'exercer un effort de compression élastique centripète sur cette dernière, de préférence à des fins thérapeutiques, par exemple pour assister ou stabiliser une structure articulaire ou musculaire.

Conformément à l'invention, l'article textile 1 comprend également un deuxième panneau 5 qui est superposé localement audit premier panneau 2, de sorte que l'article textile 1 est localement formé de deux couches, savoir une première couche formée par le premier panneau 2 et une deuxième couche, disposée sur et contre ladite première couche et formée par le deuxième panneau 5, lequel est en l'espèce de taille inférieure à celle du premier panneau 2, en particulier selon la direction longitudinale parallèle aux bords longitudinaux 20B, 20C. Conformément à l'invention, ledit deuxième panneau 5 est attaché au premier panneau 2 pour former avec ce dernier un logement 6. Les premier et deuxième panneaux 2, 5 sont donc liés mutuellement pour délimiter ensemble un volume d'accueil, formé par l'espace interstitiel entre le premier panneau 2 et le deuxième panneau 5, destiné à accueillir un élément fonctionnel indépendant 100. Comme illustré aux figures, le logement 6 est muni d'une ouverture d'accès 60 afin d'accueillir de façon amovible en son sein ledit élément fonctionnel indépendant 100, lequel forme un insert apte à être inséré, par ladite ouverture d'accès 60, dans le logement 6 ou être extrait hors dudit logement 6 par ladite ouverture 60. Le deuxième panneau 5 forme donc avec le premier panneau 2 un logement en forme d'étui, comme par exemple un fourreau, pourvu d'une ouverture d'accès 60 permettant à un utilisateur d'introduire ou de retirer à volonté, au sein ou hors dudit étui, l'élément fonctionnel 100, dont la taille et la forme sont avantageusement conjuguées à celles du logement 6.

L'élément fonctionnel indépendant 100 peut être de toute nature adaptée à la fonction recherchée. De préférence, l'élément fonctionnel indépendant 100 peut constituer une compresse thermique, formée par exemple par un sachet rempli d'un gel eutectique afin de former un « *pack gel chaud*/*froid* ». Une telle compresse thermique peut être à usage unique (jetable) ou réutilisable. Dans ce dernier cas, ladite compresse thermique est avantageusement conçue pour être « *rechargée* » thermiquement par passage dans un four à micro-ondes (lorsque l'on souhaite apporter de la chaleur à la partie du corps enserrée par l'article textile 1) ou dans un congélateur (ou un réfrigérateur) lorsque l'on souhaite refroidir la partie du corps humain concernée. Ainsi, une fois installée dans le logement 6, la compresse thérapeutique peut transférer des calories ou des frigories à la partie du corps gainée par l'article textile 1. L'invention n'est bien entendu absolument pas limitée à la mise en oeuvre d'une compresse thermique en tant qu'élément fonctionnel indépendant 100, et il est par exemple tout à fait envisageable que ledit élément fonctionnel indépendant 100 soit formé par un dispositif de diffusion d'une substance active, comme par exemple une substance médicamenteuse topique telle qu'une substance antalgique. Il est également envisageable, selon un mode de réalisation alternatif, que l'élément fonctionnel indépendant 100 soit formé par un dispositif de compression à volume variable, comprenant par exemple une vessie destinée à être gonflée (par de l'air ou un liquide) afin d'imposer localement une surpression réglable, par exemple pour comprimer une veine ou une artère. L'invention concerne d'ailleurs en tant que tel un système comprenant d'une part ledit article textile 1 selon l'invention et d'autre part ledit élément fonctionnel indépendant 100 destiné à être accueilli de façon amovible au sein du logement 6 dudit article textile 1, ledit élément fonctionnel 100 faisant partie du groupe suivant :
- compresse thermique (à usage unique ou réutilisable),
- dispositif de diffusion d'une substance active, comme par exemple une substance antalgique (à usage unique ou réutilisable),
- dispositif de compression à volume variable.

Conformément à l'invention, le deuxième panneau 5 est constitué par un deuxième tricot, qui est de préférence également, tout comme le premier tricot, un tricot à mailles cueillies, c'est-à-dire un tricot « *en trame* ». Conformément à une caractéristique importante de l'invention, ledit deuxième tricot formant le deuxième panneau 5 est composé de mailles formées par le premier fil 3, de sorte que le premier fil 3 est utilisé à la fois pour réaliser des mailles du premier tricot formant le premier panneau 2 et des mailles du deuxième tricot formant le deuxième panneau 5 superposé localement au premier panneau 2. Ainsi, le procédé de fabrication selon l'invention comprend une étape (ii) de fabrication, par tricotage dudit premier fil 3, du deuxième panneau 5 de sorte que ce dernier est superposé localement audit premier panneau 2 et est attaché à ce dernier pour former avec lui le logement 6 muni de l'ouverture d'accès 60 afin d'accueillir de façon amovible en son sein l'élément fonctionnel indépendant 100. Contrairement à l'enseignement de l'art antérieur, le deuxième panneau 5 n'est donc pas dans un premier temps rapporté contre le premier panneau 2 puis dans un deuxième temps fixé à ce dernier au moyen d'une couture, mais vient directement de matière avec le premier panneau 2, en étant fabriqué de concert avec ce dernier puisque le premier et le deuxième tricot sont réalisés par tricotage d'au moins fil commun, savoir le premier fil 3. Grâce à cette caractéristique technique, le deuxième panneau 5 est solidement attaché au premier panneau 2 sans pour autant que la fixation du deuxième panneau 5 au premier panneau 2 n'engendre le moindre inconfort puisque le deuxième panneau 5 s'étend directement dans la continuité de mailles du premier panneau 2. Un autre avantage réside dans le fait que le premier panneau 2 et le deuxième panneau 5 peuvent être réalisés dans le cadre d'une seule et même opération de tricotage. En particulier, les étapes (i) et (ii) de fabrication desdits premier et deuxième panneaux 2, 5 peuvent avantageusement être mises en oeuvre au cours d'une seule et même opération de tricotage, ce qui permet de fabriquer rapidement et à moindre coût l'article textile 1, qui présente en outre des propriétés de confort et de robustesse améliorées. Ainsi, dans le procédé de fabrication conformément à l'invention, les étapes (i), (ii) de fabrication desdits premier et deuxième panneaux 2, 5 sont effectuées, au moins en partie, simultanément, par exemple au moyen d'une seule et même tricoteuse industrielle rectiligne, comportant au moins deux fontures, et par exemple quatre fontures. Le procédé selon l'invention met ainsi avantageusement en oeuvre une unique étape de tricotage au cours de laquelle le deuxième panneau 5 est tricoté en même temps qu'une partie du premier panneau 2, selon de préférence un procédé de tricotage à plat. Ainsi, à l'issue desdites étapes de fabrication (i), (ii) desdits premier et deuxième panneaux 2, 5, ledit premier panneau 2 se présente avantageusement sous la forme d'une bande 20 (cf. figure 2) plate sur laquelle est superposé localement ledit deuxième panneau 5, ledit procédé comportant avantageusement une étape de mise en forme de ladite bande 20 dans laquelle cette dernière est refermée et assemblée sur elle-même, par liaison mutuelle des bords latéraux 20A, 20B, pour conférer audit article 1 une forme tubulaire comme illustré à la figure 1. Dans le cas où l'article 1 est conçu pour présenter seulement temporairement une forme tubulaire, le procédé comporte par exemple, en lieu et place de l'étape de mise en forme susvisée, une étape consistant à pourvoir la bande 20 de moyens de fermeture complémentaires vers ses extrémités, lesdits moyens de fermeture complémentaires se présentant par exemple sous la forme d'un système velours/crochets avec des boucles disposées vers le premier bord latéral 20A et des crochets disposés vers le deuxième bord latéral 20B, ou inversement, afin de permettre à un utilisateur de refermer en boucle la bande 20 autour du segment corporel souhaité, et ce de façon réversible.

Selon l'invention, le deuxième panneau 5 comprend un bord libre 5A qui s'étend longitudinalement entre deux extrémités 50, 51 attachées audit premier panneau 2, ledit bord libre 5A délimitant, avec le premier panneau 2, ladite ouverture d'accès 60, qui présente donc en l'espèce une forme sensiblement allongée. Cela signifie que le bord délimitant l'ouverture d'accès 60 est formé d'une part par le bord libre 5A du deuxième panneau 5 et d'autre part par la portion 200 du premier panneau 2 située sensiblement au droit dudit bord libre 5A. Dans le mode de réalisation avantageux illustré aux figures, l'étape (ii) de fabrication dudit deuxième panneau 5 est mise en oeuvre de façon que ledit deuxième panneau 5 est attaché selon sensiblement tout son pourtour audit premier panneau 2, à l'exception d'une portion localisée dudit pourtour qui forme ledit bord libre 5A délimitant, avec le premier panneau 2, l'ouverture d'accès 60. Dans le mode de réalisation illustré aux figures, le premier panneau 2 présente globalement une forme rectangulaire avec un premier côté transversal 5B et un deuxième côté transversal 5C, lesquels sont par exemple sensiblement parallèles à l'axe central X-X', et un premier côté longitudinal, qui forme en l'espèce le bord libre 5A évoqué précédemment, ainsi qu'un deuxième côté longitudinal 5D qui lui est sensiblement parallèle. Dans ce cas, le pourtour du deuxième panneau 5 est formé par le premier côté transversal 5B, le deuxième côté transversal 5C, le premier côté longitudinal 5A et le deuxième côté longitudinal 5D. Dans le mode de réalisation illustré aux figures, chacun desdits premier côté longitudinal 5D, premier côté transversal 5B et deuxième côté transversal 5C est attaché, sur sensiblement toute sa longueur, au premier panneau 2, tandis que le reste du premier panneau 2 est libre et délimite ainsi un espace interstitiel entre le premier panneau 1 et le deuxième panneau 5 qui forme le logement 6. L'ouverture d'accès 60 est quant à elle définie par le premier bord longitudinal 5A qui est attaché au premier panneau 2 uniquement en ses extrémités 50, 51, contrairement aux autres côtés 5B, 5C, 5D qui sont attachés continûment au premier panneau 2. Ainsi, dans ce mode de réalisation préférentiel, le deuxième panneau 5 présente globalement une forme rectangulaire avec les quatre côtés 5A, 5B, 5C, 5D opposés deux à deux, le deuxième panneau 5 étant avantageusement attaché continûment audit premier panneau 2 le long d'au moins trois desdits côtés 5B, 5C, 5D, le dernier côté formant ledit bord libre 5A susvisé. Un tel mode de réalisation permet de réaliser aisément, par tricotage simultané du premier panneau 2 et du deuxième panneau 5, à la fois le logement 6 et son ouverture d'accès 60, laquelle s'ouvre dès lors sur la face du premier panneau 2 contre laquelle est disposée le deuxième panneau 5. Dans le mode de réalisation illustré aux figures, le deuxième panneau 5 est disposé sur la face interne 2A, contre cette dernière, l'ouverture d'accès 60 étant dans ce cas elle aussi disposée sur la face interne 2A. Une telle conception permet de positionner l'élément fonctionnel indépendant 100 au plus près de la partie du corps humain à traiter, tout en englobant ledit élément fonctionnel indépendant au moyen du premier panneau 2, ce qui permet par exemple d'éviter, ou en tous les cas de limiter, une éventuelle déperdition thermique vers l'extérieur, lorsque l'élément fonctionnel indépendant 100 est par exemple formé par une compresse thermique. Il est cependant parfaitement envisageable, sans pour autant que l'on sorte du cadre de l'invention, que le deuxième panneau 5 soit disposé sur la face externe 2B, et que l'ouverture d'accès 60 soit dans ce cas elle aussi disposée sur la face externe 2B, ce qui permet de faciliter l'accès au logement 6 lorsque l'article textile 1 est porté par un utilisateur. Une telle configuration est cependant moins favorable en ce qui concerne le maintien de l'élément fonctionnel indépendant 100 dans le logement 6, et en ce qui concerne également les éventuelles déperditions thermiques. Ainsi, dans le mode de réalisation avantageux illustré aux figures, l'ouverture d'accès 60 est entièrement disposée du même côté que le deuxième panneau 5, ce qui évite tout enlèvement de matière à travers le premier panneau 2 pour réaliser l'ouverture d'accès 60, un tel enlèvement de matière pouvant être préjudiciable à la fonction de contention/compression/maintien élastique assuré avantageusement par le premier panneau 2. L'invention n'est cependant pas limitée à la variante préférentielle illustrée aux figures, selon laquelle l'ouverture 60 est située du même côté du premier panneau 2 que le logement 6. Il est par exemple tout à fait envisageable, sans pour autant que l'on sorte du cadre de l'invention, que le deuxième panneau 5 soit attaché selon sensiblement tout son pourtour au premier panneau 2, sans exception, de sorte que tous les côtés 5A, 5B, 5C, 5D sont reliés sensiblement continûment audit premier panneau 2, l'ouverture d'accès 60 étant dans ce cas ménagé à travers l'épaisseur premier panneau 2, entre les faces internes 2A et externe 2B. Comme illustré aux figures, le bord libre 5A s'étend de préférence sensiblement parallèlement à la direction de tricotage D du premier tricot (et du deuxième tricot), de sorte que l'ouverture d'accès 60 s'étend en l'espèce longitudinalement selon ladite direction de tricotage D, parallèlement aux rangs de mailles formant les premier et deuxième tricots.

De préférence, le deuxième panneau 5 est positionné à distance des premier et deuxième bords longitudinaux 20C, 20D de la bande 20, de sorte que le logement 6 est totalement englobé par le premier panneau 2, qui contribue ainsi au parfait maintien de l'élément fonctionnel indépendant 100 en place au sein du logement 6, grâce à l'action d'encerclement élastique du logement 6 exercée par le premier panneau 2. Dans ce mode de réalisation préférentiel, l'ouverture 60 ne s'ouvre donc pas au niveau de l'un desdits premier ou deuxième bords longitudinaux 20C, 20D, ce qui permet également de limiter la déperdition thermique (lorsque l'élément fonctionnel indépendant 100 est une compresse thermique) par l'ouverture d'accès 60 (ou de contenir la substance active émise par le dispositif de diffusion que peut constituer alternativement l'élément fonctionnel indépendant 100).

Grâce au fait que le premier panneau 2 et le deuxième panneau 5 sont tous deux formés respectivement par un premier et un deuxième tricot, qui de surcroît incluent chacun des mailles formées par le même premier fil 3, les propriétés d'extensibilité élastique de l'article textile 1 peuvent être sensiblement uniformes, en dépit de la présence localisée, sur l'une des faces (en l'espèce la face interne 2A) du premier panneau 2, du deuxième panneau 5. Afin de préserver ce caractère uniforme, le deuxième panneau 5 est avantageusement sensiblement dépourvu de tramage élastique, contrairement au premier panneau 2, afin d'éviter que l'article textile 1 ne présente localement un double tramage qui pourrait induire une modification locale de l'extensibilité élastique de l'article textile 1 et donc une éventuelle irrégularité spatiale des propriétés de compression élastique de l'article textile 1.

Cela signifie que ladite étape (ii) de fabrication dudit deuxième panneau 5 n'inclut avantageusement pas d'opération d'insertion d'un tramage élastique au sein dudit deuxième panneau 5. Ceci n'exclut pas toutefois la présence éventuelle de fils élastiques, par exemple intégrés par vanisage, dans le deuxième panneau 5, pour conférer une certaine souplesse à ce dernier. Il est également éventuellement envisageable, dans un mode de réalisation non revendiqué, que le deuxième panneau 5 puisse inclure également, comme le premier panneau 2, un tramage élastique, afin par exemple de bénéficier localement, au niveau du logement 6, d'un comportement élastique différent du fait de la superposition du tramage élastique du premier panneau 2 et du tramage élastique du deuxième panneau 5.

Selon l'invention, il est revendiqué que le bord libre 5A formant le bord de l'ouverture d'accès 60 soit contraint élastiquement, par exemple au moyen d'un ou plusieurs fils élastiques insérés au niveau et le long dudit bord libre 5A. Cette mesure technique vise en particulier à conférer audit bord libre 5A une faculté d'extensibilité élastique évitant au bord libre 5A de bâiller ou de flotter de façon relâchée, ce qui pourrait favoriser un échappement intempestif de l'élément fonctionnel indépendance 100 hors du logement 6 et/ou occasionner la formation de plis, source d'inconfort. Comme illustré aux figures, le fil élastique 4 formant le tramage élastique du premier panneau 2 est également associé au bord libre 5A pour conférer à ce dernier des propriétés d'élasticité longitudinale. Ainsi, dans ce mode de réalisation selon l'invention, le fil élastique 4 est à la fois utilisé pour former les trames élastiques du premier panneau 2, afin de permettre à ce dernier d'assurer de façon adéquate une fonction de compression ou de contention élastique, et pour conférer au bord libre 5A délimitant l'ouverture d'accès 60 une extensibilité élastique permettant d'éviter de recourir à des moyens particuliers de fermeture de l'ouverture d'accès 60 pour maintenir l'élément fonctionnel indépendant 100 au sein du logement 6. Grâce à cette caractéristique, selon laquelle le fil élastique 4 du tramage élastique du premier panneau 2 est utilisé pour contraindre élastiquement le bord libre 5A, tout en étant par ailleurs avantageusement absent du reste du deuxième panneau 5 pour les raisons déjà exposées dans ce qui précède, la construction de l'article 1 est extrêmement simple, fiable et robuste, et ne nécessite en particulier pas d'introduire des fils supplémentaires pour travailler le bord libre 5. Cette opération d'association du fil élastique 4 au bord libre 5A peut en outre être effectuée dans le cadre de l'opération de tricotage conjointe des premier et deuxième panneaux 2, 5. Le procédé selon l'invention comprend donc avantageusement une étape d'association du fil élastique 4 au bord libre 5A pour conférer à ce dernier des propriétés d'élasticité longitudinale, ladite étape d'association susvisée étant avantageusement mise en oeuvre au moyen de la même tricoteuse industrielle rectiligne avantageusement utilisée pour fabriquer les premier et deuxième panneaux 2, 5.

Avantageusement, ledit premier tricot formant le premier panneau 2 est plus épais que le deuxième tricot 5 formant le deuxième panneau 5. Cette caractéristique technique, lorsqu'elle est par exemple combinée à la caractéristique selon laquelle le logement 6 est disposé sur la face interne 2A, permet en particulier de faciliter d'éventuels transferts thermiques, à travers le deuxième panneau 5, entre l'élément fonctionnel indépendant 100 et la partie du corps humain contre laquelle le deuxième panneau 5 vient en contact lorsque le premier panneau 2 entoure la partie du corps humain en question. La mise en oeuvre d'un premier tricot d'épaisseur plus importante pour former le premier panneau 2 permet en outre de limiter la déperdition thermique vers l'extérieur. Dans le même but, le premier tricot 1 est avantageusement moins ajouré que le deuxième tricot, afin de faciliter les échanges thermiques et/ou la diffusion de substance active à travers le deuxième panneau 5.

Ces différences d'épaisseur et de « *degré de porosité* » des premier et deuxième tricots peuvent être obtenues en jouant sur le liage et/ou sur la nature du ou des fil(s) tricoté(s) pour former les premier et deuxième tricots. Avantageusement, le premier tricot formant le premier panneau 2 est majoritairement réalisé par tricotage dudit premier fil 3 selon au moins un premier liage, par exemple un liage de type tricot trame à base côte (par exemple piqué toutes aiguilles trois chutes tramé). Comme cela sera décrit plus en détails dans ce qui suit, le premier tricot peut être réalisé par tricotage d'un ou plusieurs fils selon différents liages, de sorte que le premier tricot présente dans ce cas différentes structures de maille. Avantageusement, le deuxième tricot formant le deuxième panneau 5 est quant à lui majoritairement, et de préférence entièrement, réalisé par tricotage dudit premier fil 3 selon au moins un deuxième liage différent dudit premier liage mis en oeuvre pour former le premier tricot. Par exemple, le deuxième tricot est réalisé par tricotage dudit premier fil 3 en mailles Jersey (par exemple en mailles jersey déjaugées), afin d'obtenir un deuxième panneau 5 plus fin et plus ajouré que le premier panneau 2. Ledit deuxième liage est avantageusement choisi pour conférer au deuxième panneau 5 une extensibilité suffisante pour que la présence du deuxième panneau 5 sur la face interne du premier panneau 2 n'entrave pas l'extensibilité élastique de ce dernier, de façon à conserver ainsi des propriétés de compression élastique optimale.

Comme illustré aux figures, le logement 6 constitue une poche formée par un panneau avant et un panneau arrière 7 mutuellement attachés en périphérie, ledit panneau avant étant formé par ledit deuxième panneau 5, tandis que le panneau arrière 7 est formé par une partie dudit premier panneau 2. Le panneau arrière 7 présente avantageusement, comme illustré aux figures, une forme et des dimensions sensiblement conjuguées à celles du panneau avant formé par le deuxième panneau 5. En l'espèce, le panneau arrière 7 présente ainsi une forme sensiblement rectangulaire et est lié le long de trois de ses bords au panneau avant, le long des bords 5B, 5C, 5D de ce dernier. Le premier panneau 2 est ainsi formé par ledit panneau arrière 7, ainsi que par un panneau principal 8 qui entoure au moins en partie ledit panneau arrière 7. Ainsi, dans l'exemple illustré aux figures, le panneau arrière 7 est entouré latéralement par le panneau principal 8, c'est-à-dire que le panneau principal 8 présente en l'espèce sensiblement une forme de bande rectangulaire avec un évidement rectangulaire rempli par le panneau arrière 7 (voir en particulier la figure 3). Les mailles du premier tricot qui constituent ledit panneau principal 8 sont formées par ledit premier fil 3 ainsi que par un deuxième fil 9, tandis que les mailles du premier tricot qui constituent ledit panneau arrière 7 sont formées par ledit deuxième fil 9. Cela signifie que l'étape de fabrication (i) dudit premier panneau 2 comprend:
- une opération de fabrication dudit panneau principal 8 par tricotage du premier fil 3 et dudit deuxième fil 9,
- et une opération de fabrication dudit panneau arrière 7 par tricotage dudit deuxième fil 9.

Selon l'invention, et comme illustré aux figures, le panneau principal 8 est obtenu par tricotage à la fois du premier fil 3 et du deuxième fil 9 de façon à former des rangs de mailles alternativement constitués du premier fil 3 et du deuxième fil 9 et des colonnes de mailles dont les mailles sont alternativement constituées du premier fil 3 et du deuxième fil 9, par exemple dans la proportion de une maille sur deux comme illustré à la figure 5. Selon ce même exemple de réalisation, le panneau arrière 7 est obtenu par tricotage du deuxième fil 9 uniquement, tandis que le deuxième panneau 5 est lui obtenu par tricotage du premier fil 3 uniquement. Ainsi, dans le mode de réalisation préférentiel illustré aux figures, les mailles formant le panneau arrière 7 sont exclusivement constituées par le deuxième fil 9, tandis que les mailles constituant le deuxième panneau 5, qui est superposé au panneau arrière 7, sont exclusivement formées par le premier fil 3.

Cela signifie que lors de la fabrication de l'article textile 1 conforme aux figures, les premier et deuxième fils 3, 9 sont tout d'abord tricotés ensemble, avec insertion du tramage élastique, pour former la partie du premier panneau 2 correspondant au panneau principal 8, puis que chaque fil 3, 9 est ensuite tricoté séparément, et de préférence sensiblement simultanément, pour former au cours d'une même opération de tricotage respectivement le panneau arrière 7 (pour deuxième fil 9) et le deuxième panneau 5 (pour le premier fil 3). Le tramage élastique évoqué précédemment est quant à lui uniquement inséré dans la structure de mailles du panneau arrière 7 et associé au bord libre 5A comme revendiqué. Une fois le tricotage des panneau arrière 7 et deuxième panneau 5 terminés, les deux fils 3, 9 sont à nouveau tricotés ensemble selon par exemple le liage illustré à la figure 5, pour former le reste du panneau principal 8, au sein duquel est inséré le tramage élastique. Ainsi, dans le mode de réalisation illustré aux figures, les rangs de mailles du deuxième panneau 5 s'étendent respectivement dans la continuité de chacun des rangs de mailles du panneau principal 8, à la différence près qu'ils sont tous exclusivement formés par le premier fils 3.

Les colonnes de mailles formant le deuxième panneau 5 s'étendent quant à elles dans la continuité des colonnes de mailles du panneau principal 8, à la différence près qu'elles sont uniquement formées, dans l'exemple illustré aux figures, par le premier fil 3.

En outre, afin de favoriser les échanges et transferts, en particulier thermiques, à travers le deuxième panneau 5, chaque colonne du panneau principal 8 s'étendant au droit du deuxième côté longitudinal 5D ne se prolonge pas systématiquement par une colonne de mailles du deuxième panneau 5, afin d'obtenir un deuxième tricot plus aéré. Par exemple, deux mailles sur trois du rang de mailles formant le deuxième côté longitudinal 5D forme la base d'une colonne de mailles du deuxième tricot formant le deuxième panneau 5.

De préférence, lesdits premier et deuxième fils 3, 9 sont sensiblement identiques, et sont par exemple des fils de polyamide. Il est cependant tout à fait envisageable que les premier et deuxième fils 3, 9 soit différents, sans pour autant que l'on sorte du cadre de l'invention. Il est par exemple envisageable de mettre en oeuvre un premier fil 3 plus fin que le deuxième fil 9.

En définitive, l'invention permet d'obtenir très simplement un article textile 1 de forme sensiblement tubulaire obtenu à partir d'un panneau 2 de compression élastique entièrement tricoté, avec une poche intégrée sans couture audit panneau 2, ce qui permet une efficacité (notamment en matière de transfert thermique), un confort et une esthétique particulièrement améliorés.

### POSSIBILITE D'APPLICATION INDUSTRIELLE

L'invention trouve son application industrielle dans la conception, la fabrication et l'utilisation d'articles textiles.

## Revendications

1. - Article textile (1) comprenant un premier panneau (2) destiné à entourer une partie d'un corps humain ou animal, ledit premier panneau (2) étant constitué par un premier tricot qui est composé de mailles et qui inclut un tramage élastique pour permettre audit article (1) d'enserrer élastiquement ladite partie du corps humain ou animal, ledit article textile (1) comprenant un deuxième panneau (5) superposé localement audit premier panneau (2) et attaché à ce dernier pour former avec ledit premier panneau (2) un logement (6) qui est muni d'une ouverture d'accès (60) afin d'accueillir de façon amovible en son sein un élément fonctionnel indépendant (100), ledit logement (6) constituant une poche formée par un panneau avant et un panneau arrière (7) mutuellement attachés en périphérie, ledit panneau avant étant formé par ledit deuxième panneau (5), tandis que ledit premier panneau (2) est formé par ledit panneau arrière (7) ainsi que par un panneau principal (8) qui entoure au moins en partie ledit panneau arrière (7), les mailles dudit premier tricot qui constituent ledit panneau principal (8) étant formées par un premier fil (3) ainsi que par un deuxième fil (9), tandis que les mailles du premier tricot qui constituent ledit panneau arrière (7) sont formées par ledit deuxième fil (9), ledit deuxième panneau (5) étant constitué par un deuxième tricot composé de mailles formées par ledit premier fil (3), ledit deuxième panneau (5) comprenant un bord libre (5A) qui s'étend longitudinalement entre deux extrémités (50, 51) attachées audit premier panneau (2), ledit bord libre (5A) délimitant, avec le premier panneau (2), ladite ouverture d'accès (60), l'article textile étant **caractérisé en ce que** ledit tramage élastique est formé d'au moins un fil élastique (4) qui est également associé audit bord libre (5A) pour conférer à ce dernier des propriétés d'élasticité longitudinale, ledit fil élastique (4) du tramage élastique du premier panneau (2) étant utilisé pour contraindre élastiquement le bord libre (5A), tout en étant par ailleurs absent du reste du deuxième panneau (5).

2. - Article textile (1) selon la revendication précédente **caractérisé en ce que** ledit premier panneau (2) présente une face interne (2A) destinée à se trouver en regard de ladite partie du corps humain ainsi qu'une face externe (2B) opposée, ledit deuxième panneau (5) étant disposé sur ladite face interne (2A), ladite ouverture d'accès (60) étant disposée sur ladite face interne (2A).

3. - Article textile (1) selon l'une des revendications précédentes **caractérisé en ce que** ledit deuxième panneau (5) est attaché selon sensiblement tout son pourtour audit premier panneau (2), à l'exception d'une portion localisée dudit pourtour qui forme ledit bord libre (5A) délimitant avec le premier panneau (2) ladite ouverture d'accès (60).

4. - Article textile selon l'une des revendications précédentes **caractérisé en ce que** lesdits premier et deuxième fils (3, 9) sont sensiblement identiques, et sont par exemple des fils de polyamide.

5. - Système comprenant d'une part un article textile (1) selon l'une des revendications précédentes et d'autre part un élément fonctionnel indépendant (100) destiné à être accueilli de façon amovible au sein du logement (6) dudit article textile (1), ledit élément fonctionnel (100) faisant partie du groupe suivant :
- compresse thermique,
- dispositif de diffusion d'une substance active, comme par exemple une substance antalgique,
- dispositif de compression à volume variable.

6. - Procédé de fabrication d'un article textile (1) comprenant une étape (i) de fabrication d'un premier panneau (2), par tricotage avec insertion d'un tramage élastique, ledit premier panneau (2) étant destiné à enserrer élastiquement une partie d'un corps humain ou animal, ledit procédé comprenant une étape (ii) de fabrication, par tricotage d'un premier fil (3), d'un deuxième panneau (5) superposé localement audit premier panneau (2) et attaché à ce dernier pour former avec ledit premier panneau un logement (6) qui est muni d'une ouverture d'accès (60) afin d'accueillir de façon amovible en son sein un élément fonctionnel indépendant, ledit logement (6) constituant une poche formée par un panneau avant et un panneau arrière (7) mutuellement attachés en périphérie, ledit panneau avant étant formé par ledit deuxième panneau (5), tandis que ledit premier panneau (2) est formé par ledit panneau arrière (7) ainsi que par un panneau principal (8) qui entoure au moins en partie ledit panneau arrière (7), ladite étape de fabrication (i) dudit premier panneau (2) comprenant:
- une opération de fabrication dudit panneau principal (8) par tricotage dudit premier fil (3) et d'un deuxième fil (9),
- et une opération de fabrication dudit panneau arrière (7) par tricotage dudit deuxième fil (9),
ledit deuxième panneau (5) comprenant un bord libre (5A) qui s'étend longitudinalement entre deux extrémités (50, 51) attachées audit premier panneau (2), ledit bord libre (5A) délimitant, avec le premier panneau (2), ladite ouverture d'accès (60), ledit tramage élastique étant formé d'au moins un fil élastique (4), le procédé étant **caractérisé en ce qu'**il comprend une étape d'association dudit fil élastique (4) audit bord libre (5A) pour conférer à ce dernier des propriétés d'élasticité longitudinale, ledit fil élastique (4) du tramage élastique du premier panneau (2) étant utilisé pour contraindre élastiquement le bord libre (5A), tout en étant par ailleurs absent du reste du deuxième panneau (5).

7. - Procédé selon la revendication précédente **caractérisé en ce que** lesdites étapes (i), (ii) de fabrication desdits premier et deuxième panneaux (2, 5) sont effectuées, au moins en partie, simultanément.

8. - Procédé selon l'une des revendications 6 et 7 **caractérisé en ce que** ladite étape (ii) de fabrication dudit deuxième panneau (5) est mise en oeuvre de façon que ledit deuxième panneau (5) soit attaché selon sensiblement tout son pourtour audit premier panneau (2), à l'exception d'une portion localisée dudit pourtour qui forme ledit bord libre (5A).

9. - Procédé selon l'une des revendications 6 à 8 **caractérisé en ce qu'**à l'issue desdites étapes de fabrication (i), (ii) desdits premier et deuxième panneaux (2, 5), ledit premier panneau (2) se présente sous la forme d'une bande (20) sur laquelle est superposée localement ledit deuxième panneau (5), ledit procédé comportant une étape de mise en forme de ladite bande (20) dans laquelle cette dernière est refermée et assemblée sur elle-même pour conférer audit article (1) une forme tubulaire.

10. -Procédé selon l'une des revendications 6 à 9 **caractérisé en ce que** lesdites étapes (i), (ii) de fabrication desdits premier et deuxième panneaux (2, 5) sont effectuées au moyen d'une tricoteuse industrielle rectiligne.

## Patentansprüche

1. - Textilartikel (1) umfassend eine erste Bahn (2), die dazu bestimmt ist, einen Teil eines menschlichen oder tierischen Körpers zu umgeben, wobei die erste Bahn (2) aus einem ersten Gestrick gebildet ist, das aus Maschen besteht und einen elastischen Schusseintrag umfasst, um besagtem Artikel (1) zu ermöglichen, den Teil des menschlichen oder tierischen Körpers elastisch zu umschließen, wobei der Textilartikel (1) eine zweite Bahn (5) umfasst, die lokal auf der ersten Bahn (2) liegt und an letzterer befestigt ist, um mit der ersten Bahn (2) eine Umhüllung (6) zu bilden, die mit einer Zugangsöffnung (60) versehen ist, um darin ein unabhängiges Funktionselement (100) herausnehmbar aufzunehmen;
wobei besagte Umhüllung (6) eine Tasche darstellt, die aus einer vorderen Bahn und einer hinteren Bahn (7) besteht, die an der Peripherie aneinander befestigt sind, wobei die vordere Bahn durch die zweite Bahn (5) gebildet wird, während die erste Bahn (2) durch die hintere Bahn (7) sowie durch eine Hauptbahn (8) gebildet wird, welche die hintere Bahn (7) zumindest teilweise umgibt, wobei die Maschen des ersten Gestricks, welche die Hauptbahn (8) bilden, durch ein erstes Garn (3) sowie ein zweites Garn (9) gebildet sind, während die Maschen besagten ersten Gestricks, welche die Rückwand (7) bilden, durch besagtes zweites Garn (9) gebildet sind, wobei besagte zweite Bahn (5) durch ein zweites Gestrick gebildet ist, das aus Maschen besteht, die durch besagtes erstes Garn (3) gebildet sind, wobei die zweite Bahn (5) einen freien Rand (5A) aufweist, der sich in Längsrichtung zwischen zwei Enden (50, 51) erstreckt, die an der ersten Bahn (2) befestigt sind, wobei der freie Rand (5A) zusammen mit der ersten Bahn (2) besagte Zugangsöffnung (60) begrenzt, wobei besagter Textilartikel **dadurch gekennzeichnet ist, dass** der elastische Schusseintrag aus mindestens einem elastischen Faden (4) gebildet ist, der auch mit dem freien Rand (5A) verbunden ist, um diesem Längselastizitätseigenschaften zu verleihen, wobei besagter elastische Faden (4) des elastischen Schusseintrags der ersten Bahn (2) verwendet wird, um den freien Rand (5A) elastisch zusammenzuziehen, während es ansonsten im Rest der zweiten Bahn (5) nicht vorhanden ist.

2. - Textilartikel (1) nach dem vorhergehenden Anspruch, **dadurch gekennzeichnet, dass** die erste Bahn (2) eine Innenseite (2A), die dem besagten Teil des menschlichen Körpers zugewandt ist, sowie eine gegenüberliegende Außenseite (2B) aufweist, wobei besagte zweite Bahn (5) an besagter Innenseite (2A) angeordnet ist und die Zugangsöffnung (60) an besagter Innenseite (2A) angeordnet ist.

3. - Textilartikel (1) nach einem der vorhergehenden Ansprüche, **dadurch gekennzeichnet, dass** die zweite Bahn (5) im Wesentlichen entlang ihres gesamten Umfangs an der ersten Bahn (2) befestigt ist, mit Ausnahme eines lokalen Abschnitts besagten Umfangs, der besagten freien Rand (5A) bildet, der mit der ersten Bahn (2) besagte Zugangsöffnung (60) abgrenzt.

4. - Textilartikel nach einem der vorhergehenden Ansprüche, **dadurch gekennzeichnet, dass** die ersten und zweiten Garne (3, 9) im Wesentlichen identisch sind und beispielsweise Polyamid-Garne sind.

5. - System, umfassend einerseits einen Textilartikel (1) nach einem der vorhergehenden Ansprüche und andererseits ein unabhängiges Funktionselement (100), das dazu bestimmt ist, herausnehmbar in der Umhüllung (6) besagten Textilartikels (1) untergebracht zu werden, wobei das Funktionselement (100) Teil der folgenden Gruppe ist:
- Thermokompresse,
- Vorrichtung zur Diffusion eines Wirkstoffs, wie zum Beispiel einer schmerzstillenden Substanz,
- Kompressionsvorrichtung mit variablem Volumen.

6. - Verfahren zur Herstellung eines Textilartikels (1), umfassend einen Schritt (i) der Herstellung einer ersten Bahn (2) durch Stricken unter Einfügung eines elastischen Schusseintrags, wobei die erste Bahn (2) bestimmt ist, einen Teil eines menschlichen oder tierischen Körpers elastisch zu umschließen, wobei besagtes Verfahren einen Schritt (ii) der Herstellung einer zweiten Bahn (3) durch Stricken eines ersten Garns (3) umfasst, die lokal über der ersten Bahn (2) liegt und der letzteren befestigt wird, um mit der ersten Bahn eine Umhüllung (6) zu bilden, die mit einer Zugangsöffnung (60) versehen ist, um darin ein unabhängiges Funktionselement herausnehmbar aufzunehmen, wobei die Umhüllung (6) eine Tasche bildet, die von einer vorderen Bahn und einer hinteren Bahn (7) gebildet wird, die an der Peripherie aneinander befestigt sind, wobei die vordere Wand von der zweiten Bahn (5) gebildet wird, während die erste Bahn (2) von der hinteren Wand (7) sowie von einer Hauptbahn (8) gebildet wird, welche die Rückwand (7) zumindest teilweise umgibt, wobei besagter Schritt (i) der Herstellung besagter ersten Bahn (2) folgendes umfasst:
- einen Vorgang des Herstellens besagter Hauptbahn (8) durch Stricken besagten ersten Garns (3) und eines zweiten Garns (9),
- und einen Vorgang zur Herstellung besagter Rückwand (7) durch Stricken besagten zweiten Garns (9),
wobei besagte zweite Bahn (5) einen freien Rand (5A) aufweist, der sich in Längsrichtung zwischen zwei Enden (50, 51) befindet, welche an der ersten Bahn (2) befestigt sind, wobei der freie Rand (5A) zusammen mit der ersten Bahn (2) besagte Zugangsöffnung (60) begrenzt, wobei der elastische Schusseintrag aus mindestens einem elastischen Garn (4) gebildet wird, wobei das Verfahren **dadurch gekennzeichnet ist, dass** es einen Schritt des Verbindens besagten elastische Garns (4) mit dem freien Rand (5A) umfasst, um letzterem Längselastizitätseigenschaften zu verleihen, wobei besagtes elastische Garn (4) des elastischen Schusseintrags der ersten Bahn (2) verwendet wird, um den freien Rand (5A) elastisch zusammenzuziehen, während es ansonsten im Rest der zweiten Bahn (5) nicht vorhanden ist.

7. - Verfahren nach dem vorhergehenden Anspruch, **dadurch gekennzeichnet, dass** besagte Schritte (i), (ii) der Herstellung der ersten und zweiten Bahn (2, 5) zumindest teilweise gleichzeitig durchgeführt werden.

8. - Verfahren nach einem der Ansprüche 6 und 7, **dadurch gekennzeichnet, dass** besagter Schritt (ii) zur Herstellung besagter zweiten Bahn (5) so umgesetzt wird, dass die zweite Bahn (5) im Wesentlichen entlang ihres gesamten Umfangs an der ersten Bahn (2) befestigt ist, mit Ausnahme eines lokalen Abschnitts des Umfangs, welcher den freien Rand (5A) bildet.

9. - Verfahren nach einem der Ansprüche 6 bis 8, **dadurch gekennzeichnet, dass** am Ende besagter Schritte (i), (ii) der Herstellung der ersten und zweiten Bahn (2, 5) besagte erste Bahn (2) in Form eines Streifens (20) ausgebildet ist, auf welchem besagte zweite Bahn (5) lokal überlagert ist, wobei das Verfahren einen Schritt des Formens des besagten Streifens (20) umfasst, bei dem der Streifen (20) geschlossen und zusammengesetzt wird, um dem Artikel (1) eine röhrenförmige Form zu verleihen.

10. - Verfahren nach einem der Ansprüche 6 bis 9, **dadurch gekennzeichnet, dass** besagte Schritte (i), (ii) der Herstellung der ersten und zweiten Bahn (2, 5) mit Hilfe einer industriellen Flachstrickmaschine durchgeführt werden.

## Claims

1. A textile item (1) comprising a first panel (2) intended to surround a portion of a human or animal body, said first panel (2) being formed by a first knit which is composed of stitches and which includes an elastic inlay to enable said item (1) to elastically clasp said portion of the human or animal body, said textile item (1) comprising a second panel (5) locally superimposed over said first panel (2) and attached to the latter to form with said first panel (2) a compartment (6) which is provided with an access opening (60) in order to removably receive an independent functional element (100) therein, said compartment (6) forming a pocket formed by a front panel and a rear panel (7) mutually attached at the periphery, said front panel being formed by said second panel (5), whereas said first panel (2) is formed by said rear panel (7) as well as by a main panel (8) which at least partially surrounds said rear panel (7), the stitches of said first knit which form said main panel (8) being formed by a first thread (3) as well as by a second thread (9), whereas the stitches of the first knit which form said rear panel (7) are formed by said second thread (9), said second panel (5) being formed by a second knit made up of stitches formed by said first thread (3), said second panel (5) comprising a free edge (5A) which extends longitudinally between two ends (50, 51) attached to said first panel (2), said free edge (5A) delimiting, with the first panel (2), said access opening (60), the textile item being **characterized in that** said elastic inlay is formed of at least one elastic thread (4) which is also associated to said free edge (5A) to confer longitudinal elasticity properties on the latter, said elastic thread (4) of the elastic inlay of the first panel (2) being used to elastically constrain the free edge (5A), while being, at the same time, absent from the rest of the second panel (5).

2. The textile item (1) according to the preceding claim, **characterized in that** said first panel (2) has an inner face (2A) intended to face said portion of the human body as well as an opposite outer face (2B), said second panel (5) being disposed over said inner face (2A), said access opening (60) being disposed over said inner face (2A).

3. The textile item (1) according to one of the preceding claims, **characterized in that** said second panel (5) is attached along substantially the entire perimeter thereof to said first panel (2), except for a localized portion of said perimeter which forms said free edge (5A) delimiting said access opening (60) with the first panel (2).

4. The textile item according to one of the preceding claims, **characterized in that** said first and second threads (3, 9) are substantially identical and consist, for example, of polyamide threads.

5. A System comprising, on the one hand, a textile item (1) according to one of the preceding claims and, on the other hand, an independent operating element (100) intended to be removably received within the compartment (6) of said textile item (1), said functional element (100) belonging to the following group:
- a thermal compress,
- a device for diffusing an active substance, like for example an antalgic substance,
- a variable-volume compression device.

6. A method for manufacturing a textile item (1) comprising a step (s) of manufacturing a first panel (2), by knitting with insertion of an elastic inlay, said first panel (2) being intended to elastically clasp a portion of a human or animal body, said method comprising a step (ii) of manufacturing, by knitting a first thread (3), a second panel (5) locally superimposed to said first panel (2) and attached to the latter to form with said first panel a compartment (60) which is provided with an access opening (60) in order to removably receive an independent functional element therein, said compartment (6) forming a pocket formed by a front panel and a rear panel (7) mutually attached at the periphery, said front panel being formed by said second panel (5), whereas said first panel (2) is formed by said rear panel (7) as well as by a main panel (8) which at least partially surrounds said rear panel (7), said step (i) of manufacturing said first panel (2) comprising:
- an operation of manufacturing said main panel (8) by knitting said first thread (3) and a second thread (9),
- and an operation of manufacturing said rear panel (7) by knitting said second thread (9),
said second panel (5) comprising a free edge (5A) which extends longitudinally between two ends (50, 51) attached to said first panel (2), said free edge (5A) delimiting, with the first panel (2), said access opening (60), said elastic inlay being formed of at least one elastic thread (4), the method being **characterized in that** it comprises a step of associating said elastic thread (4) to said free edge (5A) to confer longitudinal elasticity properties on the latter, said elastic thread (4) of the elastic inlay of the first panel (2) being used to elastically constrain the free edge (5A), while being, at the same time, absent from the rest of the second panel (5).

7. The method according to the preceding claim, **characterized in that** said steps (i), (ii) of manufacturing said first and second panels (2, 5) are performed, at least in part, simultaneously.

8. The method according to one of claims 6 and 7, **characterized in that** said step (ii) of manufacturing said second panel (5) is implemented so that said second panel (5) is attached along substantially the entire perimeter thereof to said first panel (2), except for a localized portion of said perimeter which forms a free edge (5A).

9. The method according to one of claims 6 to 8, **characterized in that**, upon completion of said manufacturing steps (i), (ii), said first and second panels (2, 5), said first panel (2) is in the form of a strip (20) over which said second panel (5) is locally superimposed, said method including a step of shaping said strip (20) in which the latter is closed and assembled on itself to confer a tubular shape on said item (1).

10. The method according to one of claims 6 to 9, **characterized in that** said steps (i), (ii) of manufacturing said first and second panels (2, 5) are performed by means of a rectilinear industrial knitting machine.
